# EUROPEAN PATENT APPLICATION

(11) **EP 2 003 113 A1**
(43) Date of publication of application: **17.12.2008**
(21) Application number: 07740213.9
(22) Date of filing: 29.03.2007
(51) Int. Cl.: C07C 51/60, C07C 61/15, C07C 231/02, C07C 233/63, C07B 53/00, C07B 61/00

(54) **PROCESS FOR PRODUCTION OF CARBOXYLIC ACID CHLORIDE COMPOUND**

(30) Priority: 31.03.2006 JP 2006098389
(71) Applicant: Ajinomoto Co., Inc., Tokyo 104-8315 (JP)
(72) Inventor: ISHIKAWA, Tatsuya, Yokkaichi-shi, Mie 510-0885 (JP); KONDOU, Shouichi, Yokkaichi-shi, Mie 510-0885 (JP); TAKAHASHI, Satoji, Yokkaichi-shi, Mie 510-0885 (JP)
(74) Representative: Nicholls, Kathryn Margaret
(86) International application number: PCT/JP2007/056775
(87) International publication number: WO 2007/119580

(57) **Abstract**

The present invention discloses methods for producing a carboxylic acid chloride compound which comprises the step of reacting an alkyl-substituted cyclohexyl carboxylic acid with a chlorinating agent in the presence of a specific urea compound. According to this invention, it is possible to produce a carboxylic acid chloride compound which has high reaction speed, and whose product has high purity or high yield. Thus produced carboxylic acid chloride compound is useful as an intermediate for producing D-phenylalanine derivatives which are used as agents for treating diabetes.

## Description

### Technical Field of the Invention

The present invention relates to methods for producing carboxylic acid chloride compounds which are useful as an intermediate for producing D-phenylalanine derivatives which have the hypoglycemic action and are used as agents for treating diabetes. The present invention also relates to the production of D-phenylalanine derivatives using the carboxylic acid chloride compound(s).

### Background of the Invention

Patent Literature 1 has already disclosed that D-phenylalanine derivatives including N-(trans-4-isopropylcyclohexylcarbonyl) -D-phenylalanine (nateglinide) have the hypoglycemic action and are useful as agents for treating diabetes.

Further, Patent Literature 2 discloses methods for synthesizing phenylalanine derivatives which include Schotten-Baumann reaction comprising the step of reacting trans-4-isopropylcyclohexanecarbonyl chloride (hereinafter referred to as ICCC) with phenylalanine (Phe). According to it, nateglinide can be synthesized as follows: 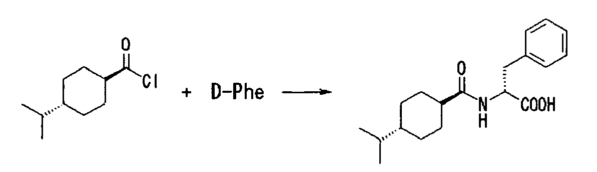 Namely, carboxylic acid chlorides including ICCC are useful as an intermediate of D-phenylalanine derivatives which are useful as agents for treating diabetes.

There are various known methods for synthesizing trans-4-isopropylcyclohexanecarbonyl chloride (ICCC) which is used as a raw material in the above synthesis of nateglinide. Patent Literature 3 discloses the following method comprising the step of making phosphorus chlorides such as phosphorous pentachloride and phosphorous trichloride or thionyl chloride act on trans-4-isopropylcyclohexanecarboxylic acid (hereinafter referred to as ICC), which is a corresponding carboxylic acid:

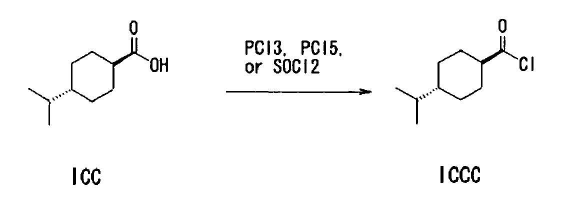

However, it has still been desired to develop methods for producing carboxylic acid chloride compounds including ICCC, which are more excellent, and, for example, have higher reaction speed, and whose products have higher purity or higher yield.
- Patent Literature 1:: JP-B 4-15221
- Patent Literature 2:: WO02/32853
- Patent Literature 3:: JP-A 7-17899

### Disclosure of the Invention

The object of the present invention is to provide methods for producing a carboxylic acid chloride compound which has high reaction speed, and whose product has high purity or high yield.

The further object of the present invention is to provide methods for effectively producing D-phenylalanine derivatives such as nateglinide, which comprise the above method for producing a carboxylic acid chloride compound.

The inventors thoroughly studied to solve the above problems and found that the problems can be solved by the method comprising the step of reacting a carboxylic acid compound with a chlorinating agent such as thionyl chloride in the presence of a compound having a specific urea structure, since the compound having a specific urea structure acts as a catalyst of the chlorination. The present invention has been completed based on this finding.

Namely, the present invention provides a method for producing a carboxylic acid chloride compound of the following formula (III), which comprises the step of reacting a carboxylic acid compound of the formula (I): 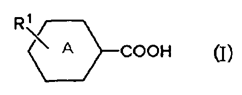 wherein Ring A represents a cyclohexane ring or a benzene ring; and R¹ represents an alkyl group having 1 to 6 carbon atoms,
with a chlorinating agent in the presence of a urea compound of the formula (II): 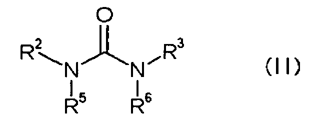 wherein R², R³, R⁵ and R⁶ each independently represents a hydrogen atom or an alkyl group having 1 to 3 carbon atoms; and R⁵ and R⁶ may bind to each other and, in such a case, -R⁵-R⁶- represents an ethylene, trimethylene, or tetramethylene group,
to synthesize said carboxylic acid chloride compound of the formula (III): 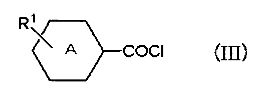 wherein Ring A and R¹ are the same as those in the above formula (I).

The present invention also provides a method for producing a D-phenylalanine derivative of the following formula (V), which comprises the steps of producing the carboxylic acid chloride compound of the formula (III) in accordance with the above production method: 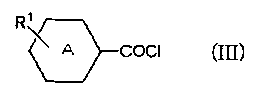 wherein Ring A and R¹ are the same as those in the above formula (I); and then reacting a D-phenylalanine compound of the formula (IV) therewith: 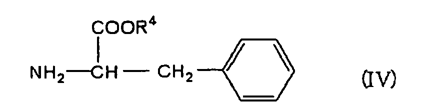 wherein R⁴ represents a hydrogen atom, an alkyl group having 1 to 3 carbon atoms or a benzyl group,
to synthesize said D-phenylalanine derivative of the formula (V): 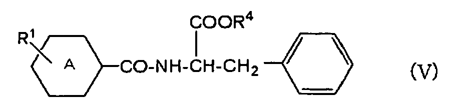 wherein Ring A, R¹ and R⁴ are the same as those mentioned in above.

According to the present invention, a carboxylic acid chloride compound which is an objective substance can be promptly obtained since the reaction quickly proceeds by using a specific urea compound.

Further, according to the preferable embodiments of the present invention, there is the advantage that, even in the case of a solid carboxylic acid compound, the carboxylic acid compound promptly changes into a liquid after the reaction starts, and therefore, it becomes easier to handle the compound such as stirring and to control the reaction. Besides, when thionyl chloride is used as a chlorinating agent, it is possible to lessen the usage amount thereof, and therefore, it is economical and good for the environment, and also makes it easier to remove thionyl chloride. Further, the carboxylic acid chloride compounds obtained by the present invention have fewer impurities such as geometric isomers.

### Best Mode for Carrying out the Invention

In the carboxylic acid compound of the formula (I) used in the present invention, Ring A represents a cyclohexane ring or a benzene ring, and a cyclohexane ring is preferable. R¹ is a straight or branched alkyl group having 1 to 6 carbon atoms, preferably an alkyl group having 2 to 5 carbon atoms, more preferably a branched alkyl group having 3 to 5 carbon atoms, and particularly preferably an isopropyl group. R¹ may be located at the o-, m- or p-position to the carboxyl group, and preferably at the p-position thereto. A particularly preferable example of the carboxylic acid compound of the formula (I) is trans-4-isopropylcyclohexanecarboxylic acid.

The present invention comprises the step of using the urea compound of the formula (II) as a catalyst when reacting the carboxylic acid compound of the formula (I) with the chlorinating agent such as thionyl chloride. Examples of the urea compound of the formula (II) include urea itself, dialkylurea, trialkylurea and tetraalkylurea, and tetraalkylurea is preferable. It is particularly preferable that, in the formula (II), R², R³, R⁵ and R⁶ each independently represents an alkyl group having 1 to 3 carbon atoms; and R⁵ and R⁶ may bind to each other and, in such a case, -R⁵-R⁶- represents an ethylene, trimethylene, or tetramethylene group. Especially, it is preferable to use 1,3-dialkyl-2-imidazolidinone wherein, in the formula (II), R⁵ and R⁶ are binding to each other and -R⁵-R⁶- is an ethylene group, and further more preferable to use 1,3-dimethyl-2-imidazolidinone (DMI) wherein both R² and R³ are a methyl group in addition to the above conditions. Since DMI is relatively stable in a strong acid, has thermostability and generates fewer decomposed materials, it is preferable in terms of control of impurities as well as the catalyst activity. Meanwhile, in the formula (II), both R² and R³ may be a hydrogen atom or a methyl group and both R⁵ and R⁶ may be a hydrogen atom, or all of R², R³, R⁵ and R⁶ may be a methyl group.

Examples of the chlorinating agent used in the present invention include at least one kind of the chlorinating agent selected from the group consisting of thionyl chloride, oxalyl chloride, phosgene, phosphorous pentachloride, phosphorous trichloride and phosphoryl chloride. Thionyl chloride is preferable among them.

When the chlorinating agent is thionyl chloride, 0.8 to 5 mol of thionyl chloride is preferably industrially used per 1 mol of the carboxylic acid compound of the formula (I), more preferably 0.8 to 2 mol thereof, and further more preferably 1 to 1.2 mol thereof is used. The urea compound of the formula (II) is preferably used in a catalytic amount thereof. For example, 0.001 to 5 parts by weight of the urea compound is preferably used per 100 parts by weight of the carboxylic acid compound of the formula (I), more preferably 0.01 to 3 parts by weight thereof, and particularly preferably 0.01 to 1 parts by weight thereof is used. Meanwhile, the urea compound may be used in larger amounts as a solvent.

This chlorination is preferably conducted without any solvent, but can be conducted with a solvent(s). It is preferable that the reaction is conducted at the temperature ranging from room temperature to around 80°C, and more preferably from 30 to 60°C. Further, it is preferable to conduct the reaction under atmospheric pressure; and after the reaction completes, to remove acidic gases such as sulfur dioxide and chlorine gas under reduced pressure, each of which is dissolved in a reaction solution. It is further more preferable to remove unreacted thionyl chloride under reduced pressure.

When the chlorinating agent is oxalyl chloride, phosgene, phosphorous pentachloride, phosphorous trichloride, phosphoryl chloride or the like, each can be used in the present invention under the same conditions as those of thionyl chloride and, if necessary, making changes to said conditions, which would be easy for those skilled in the art.

In the present invention, the carboxylic acid chloride compound of the formula (III) is particularly preferably trans-4-isopropylcyclohexanecarbonyl chloride.

It is possible to produce the D-phenylalanine derivative of the formula (V) by the method comprising the steps of producing the carboxylic acid chloride compound of the formula (III) in accordance with the above production method; and converting said carboxylic acid chloride compound. More specifically, it is possible to produce nateglinide by the method comprising the steps of producing ICCC in accordance with the above production method; and converting said ICCC.

In the present invention, the D-phenylalanine derivative of the formula (V) is produced by the method comprising the steps of producing the carboxylic acid chloride compound of the formula (III) in accordance with the above production method; and reacting the D-phenylalanine compound of the formula (IV) thereto in accordance with Schotten-Baumann reaction. In the formula (IV), R⁴ is preferably a hydrogen atom. Nateglinide is a particularly preferable example of the D-phenylalanine derivative of the formula (V).

The above reaction is preferably conducted in accordance with the conditions described in Patent Literature 2 (WO02/32853). The description of Patent Literature 2 is incorporated into the present specification.

More specifically, it is preferable to react the carboxylic acid chloride compound of the formula (III) and the phenylalanine compound of the formula (IV) in a mixed solvent of an organic solvent and water with keeping said mixed solvent in the alkaline condition by using potassium hydroxide, and preferably keeping said mixed solvent in pH 12.5 or more, and more preferably pH 13.5 or more. However, since the reaction solution is sometimes colored when the pH is over 14, the value of pH needs to be taken into account if coloring is to be avoided. When controlling pH, though the value thereof may depart from the above range, there is no problem since such departure does not exert a harmful influence if temporarily. Meanwhile, the value of pH herein mentioned is an instrument reading of a pH meter with glass electrodes. Though the concentration of an aqueous solution of potassium hydroxide is not particularly limited, it is usually 2 to 50% by weight and preferably 5 to 25% by weight.

As for an organic solvent, those which mix with water are used. Examples thereof include acetone, methyl ethyl ketone, dioxane, tetrahydrofuran, acetonitrile, methanol, ethanol, propanol and isoprpanol. Acetone is particularly preferable among them.

As for the mixture ratio of the organic solvent and water, though it differs depending on a used carboxylic acid chloride and cannot be uniformly defined, it is 10:90 to 80:20 and preferably 15:85 to 40:60.

As for the temperature and the concentration of the reaction, though they also differ depending on a used acid chloride and a reaction solvent and cannot be uniformly defined, the reaction temperature is usually -5 to 25°C and preferably 0 to 15°C, and the reaction concentration is usually 1 to 20 weight% and preferably 2 to 10 weight%. The suitable conditions thereof can be determined based on yield, operability, productivity, and the like.

As for the reaction method, it is possible to adopt the method which comprises the steps of dissolving a phenylalanine compound in water by using about the same molar amount of an aqueous solution of potassium hydroxide; adding an organic solvent(s) thereto, and then adding an aqueous solution of potassium hydroxide to control pH; and adding dropwise a carboxylic acid chloride compound thereto with stirring. The drop time is preferably 15 minutes to 2 hours. The molar ratio of the phenylalanine compound and the carboxylic acid chloride compound such as trans-4-isopropylcyclohexylcarbonyl chloride used in the reaction is 0.5:1 to 2:1 and preferably 0.9:1 to 1.5:1. The concentration of the phenylalanine compound and the acid chloride compound such as trans-4-isopropylcyclohexylcarbonyl chloride in the reaction is, when each compound is within the above ratio, preferably 2 to 15wt% in terms of the concentration of the phenylalanine compound. The generated acylphenylalanine derivative can be taken out by the steps of acidifying the reaction solution with a hydrochloric acid or the like to precipitate crystals thereof; and filtering them out and washing them with water.

According to the above production methods, it is possible to easily produce the D-phenylalanine derivative such as nateglinide in high purity by Schotten-Baumann reaction, which is an industrially superior reaction.

In addition, when the D-phenylalanine derivative of the formula (V) wherein R⁴ represents an alkyl group having 1 to 3 carbon atoms or a benzyl group is obtained by the method comprising the steps of producing the carboxylic acid chloride compound of the formula (III); and then converting said compound, a D-phenylalanine derivative of the formula (V-2) can be obtained by deesterifying said D-phenylalanine derivative of the formula (V): 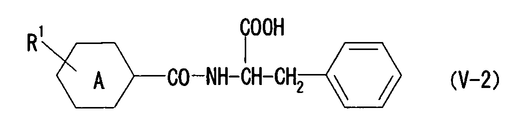 wherein Ring A and R¹ are the same as those mentioned in above.

More specifically, examples of deesterification include hydrolysis in the presence of an acid, alkali, or the like if necessary, and when R⁴ represents a benzyl group, also include catalytic hydrogenation.

Next, Examples will further illustrate the present invention.

### Examples

### Example 1 (usage of DMI: 2wt%)

0.61g (2%) of 1,3-dimethyl-2-imidazolidinone (DMI) was added to 30g (176mmol) of trans-4-isopropylcyclohexanecarboxylic acid (ICC), and 21.6g (182mmol, 1.03 equivalent) of thionyl chloride was added dropwise thereto at 40°C in 3 hours (ICC residue upon completion of the drop: 1.1%). Then, the reaction was continued for 1 hour with removing dissolved acidic gases (sulfur dioxide and hydrogen chloride) at 40°C under reduced pressure of 30kPa (ICC residue: 0.4%; thionyl chloride residue: 0.49wt%). Further, thionyl chloride was removed under 5kPa at 40 °C for 3 hours to obtain 33.6g of trans-4-isopropylcyclohexanecarbonyl chloride (ICCC) (content: 96.8wt%) as a concentrated residue. ICC residue in this ICCC was 0.3%, and thionyl chloride residue therein was less than 0.01wt%.

### Example 2 (usage of DMI: 0.05wt%)

0.014g(0.05wt%) of DMI was added to 30g (176mmol) of ICC, and 21.6g (182mmol, 1.03 equivalent) of thionyl chloride was added dropwise thereto at 40°C in 3 hours (ICC residue upon completion of the drop: 5.1%). Then, the reaction was continued for 1 hour with removing dissolved acidic gases at 40°C under reduced pressure of 30kPa (ICC residue: 1.5%; thionyl chloride residue: 0.33wt%). Further, thionyl chloride was removed under 5kPa at 40°C for 3 hours to obtain 32.9g of ICCC (content: 98.5wt%) as a concentrated residue. ICC residue in this ICCC was 1.3%, and thionyl chloride residue therein was 0.13wt%.

### Comparative Example 1 (no use of DMI)

21.6g (182mmol, 1.03 equivalent) of thionyl chloride was added dropwise to 30g of ICC (176mmol) at 40°C in 3 hours (ICC residue upon completion of the drop: 31.7%). Then, the reaction was continued for 1 hour with removing dissolved acidic gases at 40°C,under reduced pressure of 30kPa (ICC residue: 18.9%). Further, thionyl chloride was removed under 5kPa at 40°C for 3 hours to obtain 32.6g of a concentrated residue containing ICCC (content: 88.3%). ICC residue in this concentrated residue was 12.1%, and thionyl chloride residue therein was 0.39wt%.

The comparison between the result of Example 1 and that of Comparative Example 1 has clarified that, when DMI is not used (Comparative Example 1), the reaction stops in the middle though thionyl chloride is used in the same amount as that in the case of using DMI (Example 1).

### Comparative Example 2 (no use of DMI; thionyl chloride: 1.5 equivalent)

31.5g (265mmol, 1.5 equivalent) of thionyl chloride was added dropwise to 30g of ICC (176mmol) at 40°C in 3 hours (ICC residue upon completion of the drop: 15.0%). Then, the reaction was continued for 4 hours with removing dissolved acidic gases at 40°C under reduced pressure of 30kPa (ICC residue 1 hour later: 3.9%; and 4 hours later: 1.6%). Next, thionyl chloride was removed under 5kPa at 40°C for 3 hours. At that time, ICC residue in the residue containing ICCC was 1.6%, and thionyl chloride residue therein was 9.7wt%.

From the above, ICCC reaches a usable level by using thionyl chloride in an amount of 1.5 equivalent, because ICC residue is 2.0% or less. However, the residual amount of thionyl chloride is 9.7wt% and high in quantity, and therefore, it has been clarified that it does not reach a usable level of 0.2wt% or less in the same conditions as those in the case of using DMI.

Therefore, thionyl chloride was further removed under 5kPa at 40°C for 7 hours to obtain 33.4g of a concentrated residue containing ICCC. ICC residue in this ICCC was 1.4%, and thionyl chloride therein was 2.0wt%. Thus, it has been clarified that, when DMI is not used, the compound does not reach a desired quality (thionyl chloride: 0.2wt% or less) though thionyl chloride was removed in three times as long as the case of using DMI.

Meanwhile, in above Examples and Comparative Examples, a content rate of each component was calculated as follows.

### (1) ICC in the reaction solution or in the concentrated residue:

ICCC contained in the reaction solution or in the concentrated residue was derivatized to a corresponding ICC methyl ester by pretreating said reaction solution or concentrated residue. This pretreated test substance was analyzed with HPLC (detection: UV, 210nm). The area of the detected ICC was divided by the area of ICC methyl ester to calculate the content rate of ICC (ICC/ICCC%).

### (2) Thionyl chloride in the reaction solution or in the concentrated residue:

Thionyl chloride contained in the reaction solution or in the concentrated residue was derivatized to diethyl sulfite by pretreating said reaction solution or concentrated residue. This pretreated test substance was analyzed with GC. The content rate of thionyl chloride (wt%) was calculated by quantitating the detected diethyl sulfite with a standard preparation.

### (3) ICCC in the concentrated residue:

ICCC was derivatized to a corresponding amide by reacting the concentrated residue with isobutylamine in the pretreatment. This pretreated test substance was analyzed with HPLC (detection: UV, 210nm). The content rate of ICCC (wt%) was calculated by quantitating the detected amide with a standard preparation.

Table 1 shows results of Examples 1 and 2, and Comparative Examples 1 and 2.

**Table 1**

| | DMI | SOCl₂ amount | ICC amount in the reaction solution upon completion of the drop | Amount in the solution after 1h under reduced pressure of 30kPa | | Amount in the conc. residue (ICCC) after 3h under reduced pressure of 5kPa | |
|---|---|---|---|---|---|---|---|
| | | | | ICC | SOCl₂ | ICC | SOCl₂ |
| Example 1 | 2% | 1.03eq | 1.1% | 0.4% | 0.49% | 0.3% | Less than 0.01% |
| Example 2 | 0.05 % | 1.03eq | 5.1% | 1.5% | 0.33% | 1.3% | 0.13% |
| Comp. Example 1 | - | 1.03eq | 31.7% | 18.9% | - | 12.1% | 0.39% |
| Comp. Example 2 | - | 1.50eq | 15% | 3.9%* | - | 1.6% | 9.70% |

| | | | | | | | |
|---|---|---|---|---|---|---|---|
| * In Comparative Example 2, the reaction was continued for 4 hours. ICC residue of 4 hours later is 1.6%. | | | | | | | |

As for the quality of ICCC, it is required that ICC residue is 2.0% or less and thionyl chloride residue is 0.2wt% or less. However, in the case of conducting the reaction without using DMI, ICC is left when using 1.03eq of SOCl₂, and, on the other hand, the residual amount of thionyl chloride becomes larger when increasing the amount of SOCl₂. Thus, the results shown in Table 1 clarify that ICCC having a desired quality cannot be promptly obtained.

### Example 3 (1,1,3,3-tetramethylurea: 0.05%)

0.015g (0.05%) of 1,1,3,3-tetramethylurea was added to 30g (176mmol) of ICC, and 21.6g (182mmol, 1.03 equivalent) of thionyl chloride was added dropwise thereto at 40°C in 3 hours (ICC residue upon completion of the drop: 7.8%). Then, the reaction was continued for 1 hour with removing dissolved acidic gases at 40°C under reduced pressure of 30kPa (ICC residue: 2.1%). Further, thionyl chloride was removed under 5kPa at 40°C for 3 hours to obtain 32.4g of ICCC as a concentrated residue. ICC residue in this ICCC was 2.0%. Example 4 (1,3-dimethylurea: 0.05%)

0.016g (0.05%) of DMI was added to 30g (176mmol) of ICC, and 21.6g (182mmol, 1.03 equivalent) of thionyl chloride was added dropwise thereto at 40°C in 3 hours (ICC residue upon completion of the drop: 10.4%). Then, the reaction was continued for 1 hour with removing dissolved acidic gases at 40°C under reduced pressure of 30kPa (ICC residue: 3.9%). Further, thionyl chloride was removed under 5kPa at 40°C for 3 hours to obtain 30.6g of ICCC as a concentrated residue. ICC residue in this ICCC was 4.7%.

### Example 5 (urea: 0.05%)

0.016g (0.05%) of DMI was added to 30g (176mmol) of ICC, and 21.6g (182mmol, 1.03 equivalent) of thionyl chloride was added dropwise thereto at 40°C in 3 hours (ICC residue upon completion of the drop: 16.3%). Then, the reaction was continued for 1 hour with removing dissolved acidic gases at 40°C under reduced pressure of 30kPa (ICC residue: 7.1%). Further, thionyl chloride was removed under 5kPa at 40°C for 3 hours to obtain 31.7g of ICCC as a concentrated residue. ICC residue in this ICCC was 6.6% and thionyl chloride residue therein was less than 0.01wt%.

Table 2 shows results of Examples 3 to 5.

**Table 2**

| | Urea compound (0.05%) | SOCl₂ amount | ICC amount in the solution upon completion of the drop | Amount in the solution of 1h under reduced pressure of 30kPa | | Amount in the conc. residue (ICCC) of 3h under reduced pressure of 5kPa | |
|---|---|---|---|---|---|---|---|
| | | | | ICC | SOCl₂ | ICC | SOCl₂ |
| Example 3 | 1,1,3,3-tetramethylurea | 1.03eq | 7.8% | 2.1% | - | 2.0% | - |
| Example 4 | 1,3-dimethylurea | 1.03eq | 10.4% | 3.9% | - | 4.7% | - |
| Example 5 | urea | 1.03eq | 16.3% | 7.1% | - | 6.6% | less than 0.01% |

The results of Table 2 clarify that ICCC can be more promptly obtained by using a specific urea compound as compared with Comparative Example 1 in Table 1. Example 6 Production of nateglinide

143mL of water and 77mL of an aqueous solution of 10% potassium hydroxide were added to 19.3g of D-phenylalanine and dissolved. 82mL of acetone was added thereto and cooled down to about 10°C. Then, 20.0g (about 99% purity) of trans-4-isopropylcyclohexylcarbonyl chloride obtained by the same method as that of Example 2 was added dropwise thereto. Simultaneously, the pH was controlled to 13.5 to 14.0 with an aqueous solution of 10% potassium hydroxide, and the reaction was conducted to obtain an objective compound (trans-4-isopropylcyclohexylcarbonyl-D-phenylalanine).

## Claims

1. A method for producing a carboxylic acid chloride compound of the following formula (III), which comprises the step of reacting a carboxylic acid compound of the formula (I): 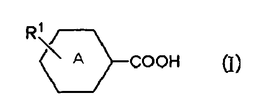 wherein Ring A represents a cyclohexane ring or a benzene ring; and R¹ represents an alkyl group having 1 to 6 carbon atoms,
with a chlorinating agent in the presence of a urea compound of the formula (II): 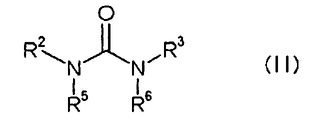 wherein R², R³, R⁵ and R⁶ each independently represents a hydrogen atom or an alkyl group having 1 to 3 carbon atoms; and R⁵ and R⁶ may bind to each other and, in such a case, -R⁵-R⁶- represents an ethylene, trimethylene, or tetramethylene group,
to synthesize said carboxylic acid chloride compound of the formula (III): 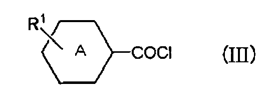 wherein Ring A and R¹ are the same as those in the above formula (I).

2. The method for producing the carboxylic acid chloride compound according to claim 1, wherein, the urea compound of the formula (II) is tetraalkylurea; and R², R³, R⁵ and R⁶ each independently represents an alkyl group having 1 to 3 carbon atoms.

3. The method for producing the carboxylic acid chloride compound according to claim 1, wherein, in the formula (II), both R² and R³ are a methyl group; and R⁵ and R⁶ bind to each other and -R⁵-R⁶- represents an ethylene group.

4. The method for producing the carboxylic acid chloride compound according to any one of claims 1 to 3, wherein 0.8 to 5 mol of the chlorinating agent is used per 1 mol of the carboxylic acid compound of the formula (I); and the urea compound of the formula (II) is used in a catalytic amount thereof.

5. The method for producing the carboxylic acid chloride compound according to any one of claims 1 to 4, wherein the reaction is conducted without any solvent and at the temperature ranging from room temperature to 80°C.

6. The method for producing the carboxylic acid chloride compound according to any one of claims 1 to 5, wherein the chlorinating agent is thionyl chloride.

7. A method for producing a D-phenylalanine derivative of the following formula (V), which comprises the steps of producing the carboxylic acid chloride compound of the formula (III) in accordance with the production method of claim 1: 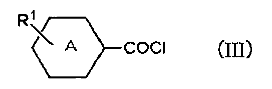 wherein Ring A and R¹ are the same as those in claim 1; and then reacting a D-phenylalanine compound of the formula (IV) with the resulting carboxylic acid chloride compound: 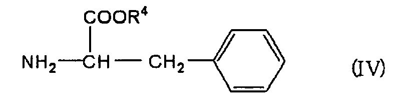 wherein R⁴ represents a hydrogen atom, an alkyl group having 1 to 3 carbon atoms or a benzyl group,
to synthesize a D-phenylalanine derivative of the formula (V): 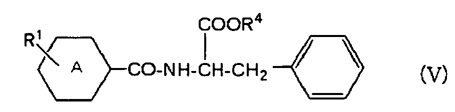 wherein Ring A, R¹ and R⁴ are the same as those mentioned above.

8. The method for producing the D-phenylalanine derivative according to claim 7, wherein R⁴ is a hydrogen atom.

9. A method for producing a D-phenylalanine derivative of the following formula (V-2), which comprises the step of deesterifying the D-phenylalanine derivative of the formula (V) obtained by the production method of claim 7, wherein R⁴ is an alkyl group having 1 to 3 carbon atoms or a benzyl group,
to synthesize said D-phenylalanine derivative of the formula (V-2): 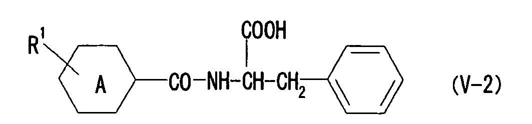 wherein Ring A and R¹ are the same as those in claim 6.

10. The production method according to any one of claims 1 to 9, wherein Ring A represents a cyclohexane ring.

11. The production method according to any one of claims 1 to 10, wherein R¹ represents a branched alkyl group having 3 to 5 carbon atoms.

12. The production method according to any one of claims 1 to 11, wherein the carboxylic acid compound of the formula (I) represents trans-4-isopropylcyclohexanecarboxylic acid.
